# EUROPÄISCHE PATENTSCHRIFT

(11) **EP 1 124 575 B1**
(45) Veröffentlichungstag und Bekanntmachung des Hinweises auf die Patenterteilung: **11.05.2005**
(21) Anmeldenummer: 99947486.9
(22) Anmeldetag: 09.10.1999
(51) Int. Cl.: A61K 39/12, C12N 7/04

(54) **VERFAHREN ZUR HERSTELLUNG EINES ANTIVIRALEN MITTELS**
METHOD FOR THE PRODUCTION OF AN ANTIVIRAL AGENT
PROCEDE DE PRODUCTION D'UN AGENT ANTIVIRAL

(30) Priorität: 28.10.1998 DE 19849641
(43) Veröffentlichungstag der Anmeldung: 22.08.2001
(73) Patentinhaber: Sonntag, Hans-Günter, 69245 Bammental (DE); Nolte, Oliver, 69181 Leimen (DE); Weiss, Hannelore, 69151 Neckargemünd (DE); Weiss, Hans-Erich, 69151 Neckargemünd (DE)
(72) Erfinder: Sonntag, Hans-Günter, 69245 Bammental (DE); Nolte, Oliver, 69181 Leimen (DE); Weiss, Hannelore, 69151 Neckargemünd (DE); Weiss, Hans-Erich, 69151 Neckargemünd (DE)
(74) Vertreter: Wagner, Jutta, Dr.
(86) Internationale Anmeldenummer: PCT/EP1999/007588
(87) Internationale Veröffentlichungsnummer: WO 2000/024420

(56) Entgegenhaltungen:
- EP-A- 0 479 280
- DD-A- 206 842
- US-A- 5 618 664
- US-A- 5 698 432
- DATABASE WPI Section Ch, Week 198141 Derwent Publications Ltd., London, GB; Class B04, AN 1981-74702D XP002133362 & JP 56 108716 A (SANWA KAGAKU KENKYUSHO CO), 28. August 1981 (1981-08-28)
- DATABASE WPI Section Ch, Week 198951 Derwent Publications Ltd., London, GB; Class B04, AN 1989-374115 XP002133363 & JP 01 279843 A (CHIBA-KEN), 10. November 1989 (1989-11-10)

## Beschreibung

Die vorliegende Erfindung betrifft ein neuartiges Verfahren zur Herstellung eines antiviralen Mittels.

Es ist bekannt, daß Viren nicht oder nur ungenügend im menschlichen oder dem Tierkörper bekämpft werden können. So ist es bis heute nicht gelungen, HIV-positive Patienten zu heilen. Dies kann darauf zurückgeführt werden, daß die Viren ihre Form individuell und über die Zeit zu ändern imstande sind, so daß vorhandene Wirkungsmechanismen ausgeschaltet werden.

Aus der US-A 5,698,432 ist bekannt, antivirale Vakzinen herzustellen, indem man kultivierte Viren vorzugsweise mit Propiolacton inaktiviert und von der Kulturflüssigkeit abtrennt, die Viren deaggregiert und die Virushülle aufbläht, vorzugsweise mit Lösemitteln und Detergentien, um anschließend mit Ethylenimin und RNAse/DNAse die virale RNA zu inaktivieren. Die so präparierten Viren werden mit Formaldehyd stabilisiert und mit Adjuvanzien zu Vakzinestandards verdünnt.

Die vorliegende Erfindung hat sich demgegenüber die Aufgabe gestellt, ein antivirales Mittel zur Verfügung zu stellen, das spezifisch zu wirken und damit die Heilungschancen wesentlich zu verbessern in der Lage ist

Die Lösung gelingt mit einem Verfahren gemäß Hauptanspruch. Vorteilhafte Ausgestaltungen dieses Verfahrens finden sich in den Unteransprüchen.

Das erfindungsgemäße Mittel stellt eine Autovakzine dar, die z. B. bei entsprechender Aufarbeitung subkutan oder peroral über die Schleimhaut verabreicht werden kann.

Durch die Behandlung patienteneigenen Blutes bei erhöhten Temperaturen in Gegenwart quervernetzender Reagenzien wie z. B. (p)Formaldehyd oder Phenol werden alle im Blut enthaltenen Proteine individuell quervemetzt und dadurch denaturiert, d. h. es wird auf das Virus bzw. das im Blut enthaltene Antigen in einer spezifischen Weise eingewirkt, was überraschenderweise nach gegebenenfalls mehrfacher Applikation zu einer Virensuppression führt.

Das entnommene Blut wird während der Abnahme und danach durch mechanische Einwirkung bzw. durch chemische Gerinnungshemmer wie z. B. EDTA, Heparin und Hirudin flüssig gehalten, um eine gute Verteilung des Vemetzungsmittels zu gewährleisten.

Durch die Denaturierungsbehandlung verfestigt sich das Blut und es formen sich die eine anti-Erreger-spezifische Immunantwort induzierenden Partikel. Das verfestigte Blut wird zur Anwendung verflüssigt, wobei man z. B. unter Rühren pyrogenfreie physiologische Kochsalzlösung einträgt.

Zur Herstellung einer (subkutan zu verabreichenden) Vakzine wird das Blut über Filter mit Porengrößen von etwa 400 µm filtriert. Das verflüssigte Mittel kann dem Patienten auch über die Schleimhäute des Mund/Rachenraumes (Gurgeln) verabreicht werden.

Das mechanische Aufrechterhalten der Fließfähigkeit des Blutes bei der Abnahme und danach, d. h. die Zerstörung von Fibrin, kann in an sich bekannter Weise durch Schütteln in Gegenwart von Glasperlen vorgenommen werden.

Soweit die zu bekämpfenden Viren vorzugsweise an die Lymphocyten gebunden sind, d.h. in den begleitenden Erythrocyten und im Serum nur in geringer Menge vorkommen, läßt sich eine Anreicherung dadurch erreichen, daß die Erythrocyten in bekannter Weise zunächst lysiert werden und Serum und lysierte Erythrocyten anschließend durch Zentrifugieren von den Lymphocyten getrennt werden. Durch Resuspendieren der Lymphocytenfraktion in physiologischer Kochsalzlösung oder in PBS (phosphate buffered saline) kann dann eine geeignete Konzentration hergestellt werden, die dann wie das Vollblut mit quervernetzenden Mitteln behandelt wird, um die erfindungsgemäße Vakzine herzustellen. Bei Viren, die in hoher Konzentration im Blutserum vorkommen (z.B. Hepatitis B- und C-Viren, deren Ursprung die Leber ist sowie andere vergleichbare Viren, die im Blut vorkommen aber ihren Ursprung nicht in den Blutzellen haben), ist das Verfahren zur Lyse der Erythrocyten nicht sinnvoll, da die Viren nach Abzentrifugation der Lymphocyten in dem die lysierten Erythrocyten enthaltenden, zu verwerfenden Überstand verbleiben.

Eine weitere Abwandlung des Protokolls stellt die Trennung der Lymphocytenfraktion von den im Blut vorkommenden Viren über 10 minütige Zentrifugation der Lymphocyten nach erfolgter Erythrocyten-Lyse dar.

Nachfolgend werden sowohl die Viren als auch die Lymphocyten in Kultur genommen. Nach erfolgreicher Virusanzucht werden die präparierten Viren zur Infektion der kultivierten Lymphocyten eingesetzt um nach einigen Tagen nach dem Herstellungsrezept der Autovakzine behandelt zu werden.

Die Viren können über routinemäßig anwendbare Präparationstechniken gereinigt werden, die Kultur der Lymphocyten setzt, ebenfalls etablierte, Methoden zur Zellkultur voraus. Die Kulturmedien sollten serumfreie Supplemente haben oder vom Patienten gewonnenes, inaktiviertes Serum als Proteinquelle enthalten. In jedem Fall ist hier besonderer Wert auf eine Sensibilitätstestung zu legen, da der Anteil körperfremder Substanzen relativ hoch liegt.

Die Methode ist z.B. für Hepatitis B Viren applikabel. Ziel dieser Behandlung bleibt es, die Autovakzine den in vivo-Verhältnissen möglichst weit anzugleichen, soll heißen, nicht nur die Viren als auslösende Erreger einer chronischen/persistierenden/rezidivierenden Infektion werden denaturiert sondern auch die Immunzellen, die mit diesen Viren in Kontakt kommen sowie die auf diesen Immunzellen exprimierten Oberflächenrezeptoren zur Antigenpräsentation. Über die Abtrennung der Lymphocyten wird wie oben eine etwas "appetitlichere" Erscheinung der Autovakzine erreicht.

Eine zusätzliche Abwandlung des Protokolls stellt die Ultraschallbehandlung der, wie oben beschrieben, gewonnenen Lymphocyten dar. Hierzu wird mit einer etablierten Ultraschallbehandlung eine Zerstörung der Zellen und damit eine Fraktionierung der Virusproteine als auch der mit Virusprotein assoziierten Oberflächenrezeptoren ermöglicht. Diese Fraktionen unterliegen in der nachfolgenden Formalin- (oder durch andere Quervernetzer erreichten) Denaturierung ebenfalls einer Quervernetzung.

Für die Quervernetzung der Proteine hat sich Formalin als besonders geeignet herausgestellt, dieses wird dem Blut in einer Menge von mindestens etwa 0,1 bis etwa 1,0 Volumenprozent in Form einer gesättigten Formalinlösung zugegeben.

Die Denaturierungstemperatur liegt bei über 50° C, vorzugsweise bei zwischen 80 und 85° C, wobei man diese Temperatur etwa 2 Stunden aufrechterhält.

Der Wirkung des erfindungsgemäßen Mittels liegt folgendes zugrunde.

Der Begriff Autovakzine beschreibt eine therapeutische Maßnahme, bei der als wirksames Agens gegen eine bakterielle chronische Infektion Erreger vom Infektionsort entnommen, je nach Fall als Reinkultur gewonnen und anschließend physikalisch und/oder chemisch verändert werden.

Im konkreten Fall bedeutet das, daß für virale Erkrankungen, bei denen der Erreger im Blut oder zeitweise im Blut befindlich ist, dieses durch simultane Anwendung von Wärme und Formaldehyd behandelt wird. Diese Behandlung des Blutes und des Antigens (des Erregers) führt zu einer Veränderung der antigenen Eigenschaften durch Denaturierung der Proteine (Wärme) bei gleichzeitiger Quervernetzung (Formalin bzw. Formaldehyd oder Para-Formaldehyd oder Phenol bzw. seine Derivate). Diese Denaturierung plus Quervernetzung führt dazu, daß das Antigen (der Erreger) dem Immunsystem in einer anderen als der nativen Weise zugänglich wird. Daraus ergibt sich, daß auch Erreger, die im nativen Zustand möglicherweise unerkannt bleiben oder eine nicht adäquate Form der Immunantwort induzieren (chronisch inflammatorischer Verlauf o. ä.), abgewehrt werden können. Hinzu kommt im Falle des Vollbluts, daß die lymphotropen Viren wie HIV auch in ihren unterschiedlichen Formen bei der Infektion der Zelle, bzw. bei der Freisetzung aus der Zelle oder in Interaktion mit bestimmten Bestandteilen/Rezeptoren der Zelle denaturiert, quervernetzt und für den Kontakt mit den antigenpräsentierenden Zellen des Immunsystems aufbereitet und zur Verfügung gestellt werden.

Diese Überlegungen werden durch umfangreiche Untersuchungen sowohl mit tierischen als auch zum Teil mit menschlichen Patienten gestützt. Größtenteils handelte es sich dabei um chronisch persistierende oder rezidivierende Infektionen. Man kann also davon ausgehen, daß das Antigen dem Immunsystem grundsätzlich zugänglich war. Nach Applikation des Antigens in denaturierter und quervernetzter Form kam es in den meisten Fällen in weniger als vier Wochen zur Heilung der Infektion. Dies kann nur durch die beschriebene veränderte Präsentationsform des Antigens durch Wärme und Formaldehyd erklärt werden.

Unsere experimentellen Daten deuten darauf hin, daß tatsächlich eine Veränderung in der Lage der Immunantwort nach Applikation der Autovakzine stattfindet. Diese Veränderung besteht, vereinfacht dargestellt, in einem Wechsel von einer inflammatorischen (Th1-) zu einer Helferzell-vermittelten (Th2-) Antwort. Aufgrund der Th2-Antwort gelingt es dem Immunsystem dann, den Erreger, der vorher zu einer chronisch inflammatorischen Situation geführt hat, zu eliminieren. Im Falle von HIV wird postuliert, daß es nach Applikation der erfindungsgemäßen Vakzine zu einer schützenden zytotoxischen T-Zell-Reaktion und gleichzeitig zu einer Veränderung in der Antikörperqualität kommt.

### Ausführungsbeispiel

100 ml Blut werden in eine sterile 500ml-Flasche eingebracht, in der sich ca. 50 sterile Glasperlen (Durchmesser 3 - 5 mm) befinden und während der Abnahme und danach geschüttelt, um das Blut zu defibrinieren. Nach dem vollständigen Eintrag wurde 10 Minuten weitergeschüttelt, das Blut blieb flüssig.

Unter weiterem Schütteln wurden 0,5 ml einer gesättigten, chemisch reinen Formalinlösung zugegeben. Danach wurde die Flasche in ein Wasserbad gestellt und die Wassertemperatur langsam auf ≥ 80° C ≤ 85° C gebracht und 2 Stunden gehalten, dabei verfestigte sich die Masse.

Anschließend wurde auf das feste (schokoiadisierte) Blut 200 ml sterile, pyrogenfreie physiologische NaCl-Lösung gegeben und die Flasche solange geschüttelt, bis die Glasperlen wieder frei waren und die Masse optisch homogen und flüssig. Danach wurde der Flascheninhalt auf ein steriles Sieb (Kantenlänge der Maschen ca. 400 µm) gegeben und mit einem sterilen Löffel durchpassiert.

Die Flasche enthält neben dem Blut 66 % phys. NaCl-Lösung sowie 0,5 ml einer gesättigten Formalin-Lösung.

Das Präparat wurde anschließend für 24 Stunden bei 37° C bebrütet.

Nach einer Sterilitätsuntersuchung wurde die Autovakzine gemäß nachstehendem Protokoll an einen Patienten abgegeben.

Für die Autovakzine-Behandlung wurde ein freiwilliger Patient ausgewählt, der nach Auskunft seines behandelnden Spezialisten eine "HIV-Infektion nach CDC Stadion C₃ mit Thrombocytopenie" aufwies, sowie weiterhin eine chronisch persistierende Hepatitis C und weitere Begleitkrankheiten, u. a. eine atypische Mycobacteriose. Aus ethischen und medizinischen Gründen war es nicht vertretbar, auf eine gleichzeitige Behandlung mit antiviralen Mitteln während der Autovakzine-Therapie zu verzichten.

Von dem Patienten lagen bezüglich der HI-Viruslast folgende Vorwerte unter Behandlung mit antiviralen Medikamenten vor:

| | |
|---|---|
| Arztbrief | 3700 / µl |
| Bestimmung 3 Monate später (vor Beginn der Autovakzine-Therapie) | 2500 / µl |

Es wurde mit Vollblut des Patienten eine Autovakzine hergestellt, wie oben dargelegt. Die Applikation erfolgte einem spezifischen Schema folgend *subcutan* und *peroral*.

Dem Patienten wurde vor Beginn der Behandlung, 7 Tage, drei Wochen und acht Wochen nach der ersten Applikation der Autovakzine heparinisiertes Vollblut entnommen. Daraus wurden nach Standardverfahren über Ficoll die Lymphocyten gereinigt, Serum abgenommen und eingefroren und mit den Lymphocyten mittels spezifischer monoklonaler Antikörper die relativen Anteile der CD4-, CD8-, CD21-und CD3- (nicht zum ersten Termin) positiven Zellen im Durchflußcytometer bestimmt. Aus dem Serum wurde nach Abschluß des Versuchs der Neopterin-Wert bestimmt.

Bei der Präparation der Lymphocyten zeigte sich vom ersten zum vierten Termin eine deutliche Zunahme der Zellausbeute pro ml Vollblut (Anmerkung: diese kann natürlicherweise schwanken), wobei der Anteil kontaminierender Zellen wie Granulocyten und Thrombocyten, die bei dieser Präparation immer anfallen, deutlich zurückging. Im Verlauf der Behandlung zeigte sich eine deutliche Zunahme der CD4-, CD8- und CD3-positiven Zellen. Die CD8-positiven Zellen stiegen dabei nach unseren Messungen deutlich über den Normwert an. CD21-positive Zellen stiegen drei Wochen nach Beginn der Autovakzine-Behandlung an, fielen dann jedoch wieder ab, blieben dabei aber immer im Normbereich. Der Index CD4/CD8 lag bei der letzten Untersuchung bei 0,5 (Norm: 1.0 - 2.3), der Anteil der CD8-positiven Zellen lag jedoch weit über dem Normwert (49,9 % gemessen durch unabhängiges Labor, 17 - 35 % Normwert), was allgemein als Zeichen einer verstärkten zytotoxischen Abwehr gegen intrazelluläre Pathogene, vorwiegend Viren, gewertet werden kann.

Der Neopterinwert (Parameter zur Verlaufskontrolle viraler sowie intrazellulärer Infektionen) im Serum war vor der Autovakzine erhöht, schwankte im Verlauf der Untersuchungen, lag nach Abschluß aber höher als zu Beginn (die Höhe des Neopterin-Spiegels wird allerdings auch durch Mycobacterien-Infektionen beeinflußt, bzw. allgemein durch inflammatorische Prozesse vom Th1-Typ, bei denen Interferon y freigesetzt wird).

Daneben zeigte der Patient physiologische Reaktionen. Etwa 30 h nach der ersten Applikation der Vakzine reagierte er mit subfebrilen Temperaturen (aber keinerlei Entzündungszeichen an der Injektionsstelle) und leichtem Durchfall, was auf eine Immunreaktion schließen läßt. Im Verlauf der darauffolgenden Wochen zeigte der Patient nach Auskunft der behandelnden Ärztin eine bleibende Gewichtszunahme sowie eine deutliche Besserung des Allgemeinzustandes.

Bestimmung der HI-Viruslast nach Abschluß der Therapie < 50 / µl (entspricht Normbereich, dieser Wert wurde durch insgesamt vier voneinander unabhängigen Untersuchungen erhoben), jedoch positiv für HIV-Provirus-DNA (Genbereich *gag*). Dieser Befund deutet lediglich an, daß provirale DNA vorhanden ist, läßt aber keine Aussage über die tatsächliche Viruslast oder den Status einer floriden Infektion zu (zur Messung der Viruslast wird die virale RNA gemessen, nach Zelltod freigesetzte DNA kann unter Umständen sehr lange nachweisbar sein).

## Patentansprüche

1. Verfahren zur Herstellung eines antiviralen Mittels, **dadurch gekennzeichnet, daß** man Viren und Antigene enthaltendes, durch Agitieren oder allgemeine Gerinnungshemmer flüssig gehaltenes Blut, in Gegenwart von Proteine quervernetzenden Mitteln wie vorzugsweise Formaldehyd, p-Formaldehyd und/oder Phenol oder Phenolderivaten, auf Temperaturen von Ober 50 °C erwärmt, das dabei verfestigte Blut verflüssigt und zur Herstellung einer Vakzine durch ein engporiges Filter passiert.

2. Verfahren nach Anspruch 1, **dadurch gekennzeichnet, daß** man das verfestigte Blut mit pyrogenfreier physiologischer Kochsalzlösung verflüssigt.

3. Verfahren nach einem der Ansprüche 1 bis 2, **dadurch gekennzeichnet, daß** man die Agitation und die Verflüssigung durch die Kochsalzlösungszugabe durch Schütteln in Gegenwart von Glasperlen durchführt.

4. Verfahren nach einem der Ansprüche 1 bis 3, **dadurch gekennzeichnet, daß** dem Blut 0,3 bis 1,0, vorzugsweise 0,5 Volumenprozent einer gesättigten Formalinlösung zugegeben werden.

5. Verfahren nach einem der Ansprüche 1 bis 4, **dadurch gekennzeichnet, daß** man das Blut in Gegenwart des quervernetzenden Mittels auf Temperaturen von 55 bis 85 °C erwärmt und diese Temperatur etwa 2 Stunden hält.

6. Verfahren nach einem der Ansprüche 1-5, **dadurch gekennzeichnet, daß** das Blut zunächst einer Erythrocytenlyse unterworfen, die Lymphocytenfraktion abzentrifugiert, in physiologischer Kochsalzlösung oder PBS resuspendiert wird und danach mit den quervemetzenden Mitteln behandelt wird.

7. Denaturierte Antigene und Viren, erhältlich nach einem der Ansprüche 1 bis 6.

8. Antivirales Mittel, enthaltend Antigene und/oder Viren erhältlich nach einem der Ansprüche 1 bis 6.

## Claims

1. Process for the preparation of an antiviral agent, **characterised in that** one warms virus- and antigen-containing blood kept liquid by agitation or general coagulation inhibitors in the presence of protein cross-linking agents, such as preferably formaldehyde, p-formaldehyde and/or phenol or phenol derivatives to temperatures of over 50°C, liquefies the thereby solidified blood and passes it, for the preparation of a vaccine, through a narrow-pored filter.

2. Process according to claim 1, **characterised in that** the solidified blood is liquefied with pyrogen-free physiological common salt solution.

3. Process according to one of claims 1 to 2, **characterised in that** one carries out the agitation and the liquefaction by the common salt solution addition by shaking in the presence of glass pearls.

4. Process according to one of claims 1 to 3, **characterised in that** to the blood are added 0.3 to 1.0, preferably 0.5 volume percent of a saturated formalin solution.

5. Process according to one of claims 1 to 4, **characterised in that** one warms the blood in the presence of the cross-linking agent to temperatures of 55 to 85°C and maintains this temperature for about 2 hours.

6. Process according to one of claims 1-5, **characterised in that** the blood is first subjected to an erythrocyte lysis, the lymphocyte fraction is centrifuged off, the blood is resuspended in physiological common salt solution or PBS and is thereafter treated with the cross-linking agents.

7. Denatured antigens and viruses obtainable according to one of claims 1 to 6.

8. Antiviral agent containing antigens and/or viruses obtainable according to one of claims 1 to 6.

## Revendications

1. Procédé de production d'un agent antiviral, **caractérisé en ce que** l'on chauffe à des températures de plus de 50°C, du sang maintenu liquide par une agitation ou des anticoagulants généraux, contenant des virus et des antigènes, en présence d'agents de réticulation de protéines comme par exemple le formaldéhyde, le p-formaldéhyde et/ou le phénol et les dérivés de phénol, on liquéfie le sang ainsi solidifié et on le fait passer à travers un filtre à pores étroits pour produire un vaccin.

2. Procédé selon la revendication 1, **caractérisé en ce que** l'on liquéfie le sang solidifié avec une solution de sérum physiologique apyrogène.

3. Procédé selon l'une quelconque des revendications 1 à 2, **caractérisé en ce que** l'on réalise l'agitation et la liquéfaction par ajout de sérum physiologique, en secouant en présence de perles de verre.

4. Procédé selon l'une quelconque des revendications 1 à 3, **caractérisé en ce que** l'on ajoute au sang, 0,3 à 1,0, de préférence 0,5% en volume d'une solution de formaline saturée.

5. Procédé selon l'une quelconque des revendications 1 à 4, **caractérisé en ce que** l'on chauffe le sang à des températures de 55 à 85°C en présence de l'agent de réticulation, et on maintient cette température pendant environ 2 heures.

6. Procédé selon l'une quelconque des revendications 1 à 5, **caractérisé en ce que** le sang est d'abord soumis à une lyse d'érythrocytes, la fraction des lymphocytes est séparée par centrifugation, remis en suspension dans du sérum physiologique ou du PBS, puis traité par les agents de réticulation.

7. Antigènes et virus dénaturés pouvant être obtenus selon l'une quelconque des revendications 1 à 6.

8. Agent antiviral, contenant des antigènes et/ou des virus, pouvant être obtenu selon l'une quelconque des revendications 1 à 6.
